# EUROPEAN PATENT SPECIFICATION

(11) **EP 2 117 539 B1**
(45) Date of publication and mention of the grant of the patent: **01.09.2010**
(21) Application number: 07821854.2
(22) Date of filing: 25.10.2007
(51) Int. Cl.: A61K 31/4162, A61P 35/00

(54) **A METHOD OF ADMINISTERING AN ANTITUMOR COMPOUND**
VERFAHREN ZUR VERABREICHUNG EINER ANTITUMORALEN VERBINDUNG
PROCÉDÉ D'ADMINISTRATION D'UN COMPOSÉ ANTI-TUMEUR

(30) Priority: 03.11.2006 EP 06123440
(43) Date of publication of application: 18.11.2009
(73) Proprietor: Nerviano Medical Sciences S.r.l., 20014 Nerviano (MI) (IT)
(72) Inventor: LAFFRANCHI, Bernard, 20080 Bubbiano (Milan) (IT); MOLL, Jurgen, 22070 Appiano Gentile (Como) (IT); BOLOGNA, Fabrina, 20010 Pogliano Milanese (Milan) (IT); COMIS, Silvia, 20080 Bubbiano (Milan) (IT)
(86) International application number: PCT/EP2007/061491
(87) International publication number: WO 2008/052931

(56) References cited:
- WO-A-2005/005427
- WO-A-2007/113212

## Description

The present invention relates in general to the field of cancer treatment and, more particularly, is related to the treatment of tumours with an aurora inhibitor.

The present invention provides the use of Compound I of the formula A: 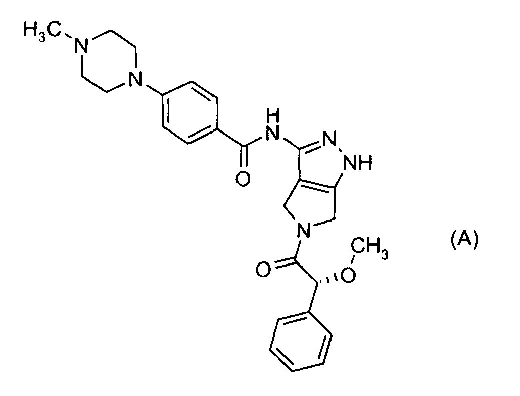 or a pharmaceutically acceptable salt thereof, for treating a mammal, including humans, suffering from tumour, **characterised in that** Compound 1 is conveniently intravenously infused employing particular schedules which allow a more efficacious treatment of tumours.

Compound 1 of formula A was described and claimed in the international patent application WO2005/005427, published on December 20, 2005.

The treatment therein described was focused on the oral route. Intravenous administration described there is very general.

We have now found that Compound I of formula A is much more efficacious when intravenously infused on days 1, 8 and 15 every 4 weeks, in one single day every one or two weeks, for three consecutive days every two weeks, for three consecutive days the first week and then twice per week from weeks 2 to 4 in cycles of 5 weeks, or for 7 consecutive days every 2 weeks.

Preferably, when intravenously infused on days 1, 8 and 15 every 4 weeks, Compound 1 of formula A is in an amount of from 100 to 850 mg/m²/day; when intravenously infused in one single day every one or two weeks, it is in an amount of from 100 to 1000 mg/m²/day and when intravenously infused for three consecutive days every two weeks, it is in an amount of from 100 to 1000 mg/m²/day.

Preferably, when intravenously infused for three consecutive days the first week and then twice per week from weeks 2 to 4 in cycles of 5 weeks, Compound I of formula A is in an amount of from 100 to 1000 mg/m²/day.

Preferably, when intravenously infused for 7 consecutive days every 2 weeks, Compound I of formula A is in an amount of from 40 to 500 mg/m²/day.

Therefore, in a first aspect, the present invention provides the use of Compound 1 of the formula A as above defined, or a pharmaceutically acceptable salt thereof, for treating a mammal, including humans, suffering from tumour, characterized in that Compound 1 is intravenously infused on days 1, 8 and 15 every 4 weeks, preferably in an amount of from 100 to 850 mg/m²/day, more preferably from 300 to 500 mg/m²/day, in one single day every one or two weeks, preferably in an amount of from 100 to 1000 mg/m²/day, more preferably from 400 to 800 mg/m²/day, for three consecutive days every two weeks, preferably in an amount of from 100 to 1000 mg/m²/day, more preferably from 200 to 800 mg/m²/day, for three consecutive days the first week and then twice per week from weeks 2 to 4 in cycles of 5 weeks, preferably in an amount of from 100 to 1000 mg/m²/day, or for 7 consecutive days every 2 weeks, preferably in an amount of from 40 to 500 mg/m²/day.

The chemical name of Compound 1 of formula A is N-{5-[(2R)-2-methoxy-2-phenytethanoyl]-1,4,5,6-tetrahydropyrrolo[3,4-c] pyrazol-3-yl}-4-(4-methylpiperazin-1-yl)benzamide, herein referred to as "Compound 1" or "Compound 1 of formula A". Pharmaceutically acceptable salts of Compound 1 of formula A include the acid addition salts with inorganic or organic acids such as, for instance, nitric, hydrochloric, hydrobromic, sulphuric, perchloric, phosphoric, acetic, trifluoroacetic propionic, glycolic, lactic, oxalic, malonic, malic, maleic, tartaric, citric, benzoic, cinnamic, mandelic, methanesulphonic, isethionic and salicylic acid.

Compound I to be used in the present invention can be prepared according to the process disclosed in WO2005/005427.

Compounds of this chemical class have revealed to be potent ATP-competitive inhibitors of Aurora kinases (FANCELLI, D., ct al. Potent and selective Aurora inhihitors identified by the expansion of a novel scaffold for protein kinase inhihition, J. Med. Chem. 2005, 48, no.8, p.3080-3084). In particular, Compound 1 has been found to display a significant inhibitory potency towards Aurora kinases (AKs) A, B and C. Aurora A has been shown to act as an oncogene since over-expression of wild type Aurora A or of a constitutive active mutant, transforms Rat and NIH 3T3 cells leading to colony formation in soft agar assays. Also NIH 3T3 cells expressing constitutively active Aurora A can grow as solid tumors when injected into *nu*/*nu* mice. When Aurora A is over-expressed in the diploid human breast cell line MCFIOA centrosome abnormalities and aneuploidy are observed. A direct role of Aurora B or C in tumorigenesis is less well documented, although Aurora B is also over-expressed in many human tumors and upon inhibition cells go into aberrant mitosis. Aurora kinases, by virtue of their roles in mitosis have been implicated in the genetic instability of tumor cells by controlling chromosomal ploidy. Some of the known substrates or interacting proteins of Aurora kinases, such as RasGAP, p53, Cdc20 or NM23-H 1 could be important mediators in malignant transformation. These properties make the aurora kinases attractive targets for anti cancer therapy.

In view of its biological activity, Compound 1 of the invention offers a new path for the development of a treatment for patient populations suffering from several tumours.

In another aspect of the present invention, there is provided a method of treating a mammal, including humans, suffering from tumour, comprising administering Compound 1 of the formula A as above defined or a pharmaceutically acceptable salt thereof to said mammal by intravenous infusion on days 1, 8 and 15 every 4 weeks, preferably in an amount of from 100 to 850 mg/m²/day, more preferably from 300 to 500 mg/m²/day, in one single day every one or two weeks, preferably in an amount of from 100 to 1000 mg/m²/day, more preferably from 400 to 800 mg/m²/day or for three consecutive days every two weeks, preferably in an amount of from 100 to 1000 mg/m²/day, more preferably from 200 to 800 mg/m²/day, for three consecutive days the first week and then twice per week from weeks 2 to 4 in cycles of 5 weeks, preferably in an amount of from 100 to 1000 mg/m²/day, or for 7 consecutive days every 2 weeks, preferably in an amount of from 40 to 500 mg/m²/day.

A further aspect of the present invention is to provide a method of preparation of a medicament for use in the tumour therapy in mammals, including humans, said medicament comprising Compound 1 of formula A as above defined, or a or a pharmaceutically acceptable salt thereof and a pharmaceutically acceptable carrier or excipient, characterized in that the medicament is intravenously infused on days 1, 8 and 15 every 4 weeks, preferably in an amount of from 100 to 850 mg/m²/day, more preferably from 300 to 500 mg/m²/day, in one single day every one or two weeks, preferably in an amount of from 100 to 1000 mg/m²/day, more preferably from 400 to 800 mg/m²/day or for three consecutive days every two weeks, preferably in an amount of from 100 to 1000 mg/m²/day, more preferably from 200 to 800 mg/m²/day, for three consecutive days the first week and then twice per week from weeks 2 to 4 in cycles of 5 weeks, preferably in an amount of from 100 to 1000 mg/m²/day, or for 7 consecutive days every 2 weeks, preferably in an amount of from 40 to 500 mg/m²/day.

Preferably, the patients to be treated according to the present invention have advanced solid tumours or hematopoietic malignant tumours.

The exact dosage range adopted will depend upon the age, body surface area (m²) and condition of the patient being treated.

Compound 1 of formula A can be administered as a continuous infusion over from 30 minutes to 24 hours, preferably from 1 to 24 hours.

Still more preferably, Compound 1 can be administered as a continuous infusion using programmable continuous infusion ambulatory pump or iv infusion bags, over a period of about 6 hours when administered on days 1, 8 and 15 every 4 weeks;
about 24, 6 or 3 hours when administered in one single day every one or two weeks or 1 to 6 hours or about 3 hours when administered for three consecutive days every 2 weeks. When Compound 1 is administered on days 1, 2, and 3 the first week, followed by two administrations per week from the 2nd to 4th weeks every 5 weeks, or for 7 consecutive days every 2 weeks, the duration of the infusions varies from 1 to 24 hours and is preferably of 1, 3 or 6 hours, even more preferably 3 hours.

The present anti-tumour agent exerts a strong anti-tumour activity on human malignant tumours, for example, on solid tumours such as gastrointestinal tumours, like colorectal cancer, gastro-ocsophageal cancer, cancer of liver and biliary tract, pancreatic cancer; prostatic cancer, testicular cancer, lung cancer, breast cancer, malignant melanoma, mesothelioma, brain tumours (such as glioma and astrocytomas), ovarian cancer, uterine cancer including cervical cancer, cancer of the head and neck, bladder cancer, Kaposi sarcoma, including AIDS-related Kaposi sarcoma, sarcomas and osteosarcoma, renal carcinoma; and hematopoietic malignant tumours such as leukaemias and lymphoma (i.e. ALL, Acute Lymphoblastic Leukaemia, CLL, Chronic Lymphocytic Leukaemia, MM, Multiple Myeloma, CML, Chronic Myeloid Leukaemia, AML, Acute Myeloid Leukaemia) including AIDS-related lymphomas.

The pharmaceutical compositions of the present invention containing Compound 1 may be formulated together with a pharmaceutically carrier or diluent. Typically they are formulated for parenteral administration, for example by dissolution in water for injection or physiological saline. Compound 1 is more preferably supplied as units of sterile solution, 15 ml vials (10 mg/ml) for injection containing 150 mg of Compound 1 as active ingredient.

Depending on the neoplastic condition, Compound 1 and the pharmaceutical compositions of the invention can be administered together with a factor which can reduce negative side effects that may arise from, or be associated with, administration of Compound 1 or pharmaceutical composition alone. Cytokines, lymphokines, growth factors, or other hematopoietic factors are useful, more particularly G-CSF.

The following examples illustrate the invention.

### Example 1

### Drug administration

### Vials containing

- 0.150 g of Compound 1,
- 15 ml of 5 % dextrose solution adjusted to pH 5 with hydrochloric acid or sodium hydroxide. The dose to be administered to the patient was put in an infusion bag containing 500 ml saline and an equal volume was then removed from the bag so that the final volume after adding the drug was always 500 ml. The resultant solution was administered intravenously in a time ranging from 3 to 24 hours. Before and after the infusion, the vein was washed with saline, 10 ml over 5'.

### Example 2

A phase I pharmacological trial was carried out to investigate the iv administration of Compound 1 given in 24 hours infusion once every two weeks in patients with refractory or resistant solid tumours, including lung, colorectal, gastro-oesophageal, cervix, Ewing, renal, head and neck, mesothelioma and cancer of unknown primary.

This was a dose escalation study. Cohorts of 3-6 patients were sequentially allotted to progressively higher dose levels (DL) of Compound I based on the number of dose limiting toxicities (DLTs) observed. As per protocol, definition of DLTs were grade (G) 4 neutropenia lasting >7 days, febrile neutropenia, neutropenic infection, any G3 or 4 non-hematological drug related toxicities during the first cycle of treatment.

Forty patients were enrolled. Seven DLs were explored (45, 90, 180, 360, 500, 650 and 580 mg/m²). Most of the patients at the 500, 650 and 580 mg/m² had G3 and 4 leucopenia/neutropenia. No G4 non-hematological toxicity was reported. Except one G3 diarrhea, pyrexia and fatigue in one patient each, all other drug related non-hematological adverse events were G1 or 2 (fatigue, anorexia, nausea, vomiting, and ejection fraction decreased). One DLT (neutropenic infection) in the 360 mg/m², 2 DLTs (neutropenic infection and febrile neutropenia) at DL 650 mg/m² and 2 DLTs at 580 mg/m² (G 3 liver enzyme increase and febrile neutropenia) were reported. The MTD has been exceeded at 650 and 580 mg/m². Nine pts showed stable diseases as best response and in 3 of them the response duration was ≥ 6 months. Inhibition of histone H3 phosphorylation induced by Compound 1 was evident in skin biopsies of patients treated at ≥ 500 mg/m². Compound 1 clearance was 0.3-0.45 L/h/kg, with a volume of distribution 2-4 times total body water and a terminal half-life of 18-30 hours. Compound 1 showed dose-proportional and time-independcnt behavior. Neutropenia was the dose-limiting toxicity for Compound 1 and easily managed. Non-hematological drug related adverse events were mild or moderate. Biomarker modulation occurred at ≥ 500 mg/m². Pharmacokinctic was linear with low inter-individual variability. The recommended dose for Phase II was 500 mg/m². Furthermore the protocol was amended to increase the dose up to 750 mg/m² with prophylactic treatment with G-CSF.

### Example 3

In a two-center phase I dose escalation trial, Compound I was administered to patients with advanced/metastatic solid tumors (colon, pancreatic, renal cell, esophageal, NSLC, Non-Small Cell Lung Cancer , ovarian carcinoma, sarcoma, other) by a 6-hours IV infusion weekly for 3 consecutive weeks, in a 28-day cycle. The infusion duration was selected to reduce the potential for Cₘₐₓ related toxicities. Forty patients were treated with doses ranging from 45 to 400 mg/m². The protocol was amended to allow the administration of the drug at days 1, 8 and 15 even in case of uncomplicated grade 3 neutropenia (frequently reported at 250 mg/m²). In order to define the recommended dose for phase II, 330 mg/m² dose level was investigated.

Uncomplicated neutropenia of short duration was the only hematological toxicity reported. Drug-related non hematological toxicities (G1/G2 diarrhea, nausea, vomiting, fatigue, anorexia and blood pressure increase) were mild or moderate.

No drug-related deaths were reported. Seven patients showed stable disease as best response, lasting more than seven months for four of them.

Furthermore, the protocol was amended to explored shorter time of infusion.

### Example 4

A phase I pharmacological study to investigate the iv administration of Compound 1 given in a 3-hour intravenous infusion for three consecutive days every two weeks in patients with hematopoietic malignancies such as relapsed/refractory acute leukemia. Ten patients have been enrolled in 4 dose levels (100, 150, 210, and 280 mg/m²/daily). This regimen was well tolerated by the patients. Dose escalation is still proceeding.

### Example 5

A phase I pharmacological study to investigate the iv administration of Compound 1 given as a 3-hour intravenous infusion every two weeks in patients with hematopoietic malignancies such as relapsed/refractory chronic lymphocytic leukemia, lymphoma and multiple myeloma already explored the administration of 200 and 300 mg/m² and dose escalation is currently ongoing at 450 mg/m².

### Example 6

A phase 1 pharmacological study to investigate the iv administration of Compound I given either as a 3-hour iv infusion daily for 7 consecutive days every 2 weeks or given once weekly over 24-hour iv infusions is currently ongoing in patients suffering from CML.

### Example 7

Two phase II studies are currently evaluating doses of 330 or 400 mg/m² given once per week as a 6-hour iv infusion in CML patients, and as 3-hour iv infusions in patients with multiple myeloma.

## Claims

1. Use of Compound 1 of the formula A: 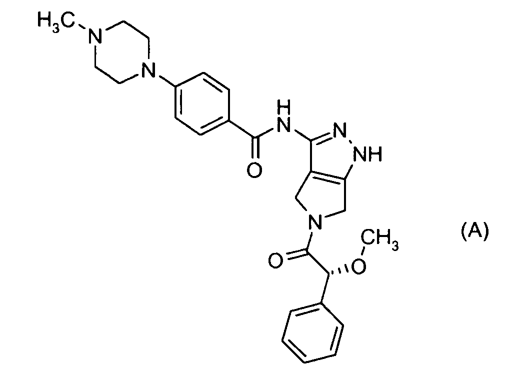 or a pharmaceutically acceptable salt thereof, and a pharmaceutically acceptable carrier or excipient, in the manufacture of a medicament for treating a mammal, including humans, suffering from advanced solid tumours or hematopoietic malignant tumours, **characterized in that** Compound 1 is intravenously infused on days 1, 8 and 15 every 4 weeks, in one single day every one or two weeks, for three consecutive days every two weeks, for three consecutive days the first week and then twice per week from weeks 2 to 4 in cycles of 5 weeks, or for 7 consecutive days every 2 weeks.

2. Use according to claim 1, **characterized in that** Compound 1 is intravenously infused on days 1, 8 and 15 every 4 weeks in an amount of from 100 to 850 mg/m²/day.

3. Use according to claim 1 **characterized in that** Compound 1 is intravenously infused in one single day every one or two weeks in an amount of from 100 to 1000 mg/m²/day.

4. Use according to claim 1 **characterized in that** Compound 1 is intravenously infused for three consecutive days every two weeks in an amount of from 100 to 1000 mg/m²/day.

5. Use according to claim 1 **characterized in that** Compound 1 is intravenously infused for three consecutive days the first week and then twice per week from weeks 2 to 4 or from weeks 2 to 5 in cycles of 5 weeks in an amount of from 100 to 1000 mg/m²/day.

6. Use according to claim 1 **characterized in that** Compound 1 is intravenously infused for seven consecutive days every 2 weeks in an amount of from 40 to 500 mg/m2/day.

7. Use according to claim 1 **characterized in that** Compound 1 is administered as a continuous infusion over a period of from 30 minutes to 24 hours.

8. Use according to claim 1 **characterized in that** Compound 1 is administered as a continuous infusion over a period of from 1 to 24 hours.

9. Use according to claim 2 **characterized in that** Compound 1 is administered as a continuous infusion over a period of about 6 hours.

10. Use according to claim 3 **characterized in that** Compound 1 is administered as a continuous infusion over a period of about 24, 6 or 3 hours.

11. Use according to claim 4 **characterized in that** Compound 1 is administered as a continuous infusion over a period of about 1 to 6 hours or about 3 hours.

12. Use according to claims 5 or 6 **characterized in that** Compound 1 is administered as a continuous infusion over a period of about 3 hours.

## Patentansprüche

1. Verwendung von Verbindung 1 mit der Formel A: 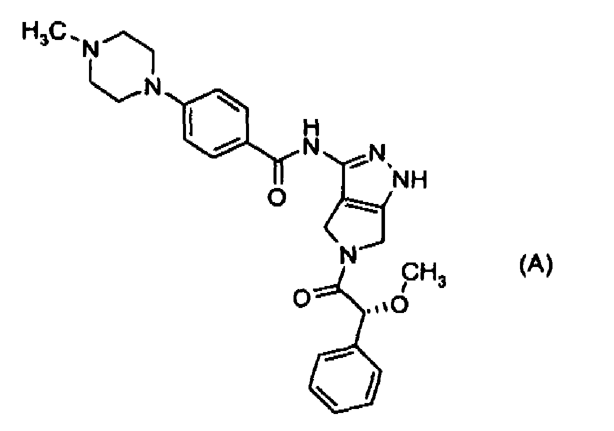 oder eines pharmazeutisch akzeptablen Salzes davon, und eines pharmazeutisch akzeptablen Trägers oder Hilfsstoffes bei der Herstellung eines Medikamentes zur Behandlung eines Säugetiers, einschließlich Menschen, welches an fortgeschrittenen soliden Tumoren oder hämatopoetischen malignen Tumoren leidet, **dadurch gekennzeichnet, dass** Verbindung 1 alle 4 Wochen an Tag 1, 8 und 15, an einem einzigen Tag alle ein oder zwei Wochen, an drei aufeinanderfolgenden Tagen alle zwei Wochen, an drei aufeinanderfolgenden Tagen in der ersten Woche und dann zweimal pro Woche von Woche 2 bis 4 in Zyklen von 5 Wochen oder an 7 aufeinanderfolgenden Tagen alle 2 Wochen intravenös infundiert wird.

2. Verwendung nach Anspruch 1, **dadurch gekennzeichnet, dass** Verbindung 1 alle 4 Wochen an Tag 1, 8 und 15 in einer Menge von 100 bis 850 mg/m²/Tag intravenös infundiert wird.

3. Verwendung nach Anspruch 1, **dadurch gekennzeichnet, dass** Verbindung 1 an einem einzigen Tag alle ein oder zwei Wochen in einer Menge von 100 bis 1000 mg/m²/Tag intravenös infundiert wird.

4. Verwendung nach Anspruch 1, **dadurch gekennzeichnet, dass** Verbindung 1 an drei aufeinanderfolgenden Tagen alle zwei Wochen in einer Menge von 100 bis 1000 mg/m²/Tag intravenös infundiert wird.

5. Verwendung nach Anspruch 1, **dadurch gekennzeichnet, dass** Verbindung 1 an drei aufeinanderfolgenden Tagen in der ersten Woche und dann zweimal pro Woche von Woche 2 bis 4 oder von Woche 2 bis 5 in Zyklen von 5 Wochen in einer Menge von 100 bis 1000 mg/m²/Tag intravenös infundiert wird.

6. Verwendung nach Anspruch 1, **dadurch gekennzeichnet, dass** Verbindung 1 an sieben aufeinanderfolgenden Tagen alle zwei Wochen in einer Menge von 40 bis 500 mg/m²/Tag intravenös infundiert wird.

7. Verwendung nach Anspruch 1, **dadurch gekennzeichnet, dass** Verbindung 1 als eine Dauerinfusion über einen Zeitraum von 30 Minuten bis 24 Stunden verabreicht wird.

8. Verwendung nach Anspruch 1, **dadurch gekennzeichnet, dass** Verbindung 1 als eine Dauerinfusion über einen Zeitraum von 1 bis 24 Stunden verabreicht wird.

9. Verwendung nach Anspruch 2, **dadurch gekennzeichnet, dass** Verbindung 1 als eine Dauerinfusion über einen Zeitraum von etwa 6 Stunden verabreicht wird.

10. Verwendung nach Anspruch 3, **dadurch gekennzeichnet, dass** Verbindung 1 als eine Dauerinfusion über einen Zeitraum von etwa 24, 6 oder 3 Stunden verabreicht wird.

11. Verwendung nach Anspruch 4, **dadurch gekennzeichnet, dass** Verbindung 1 als eine Dauerinfusion über einen Zeitraum von etwa 1 bis 6 Stunden oder etwa 3 Stunden verabreicht wird.

12. Verwendung nach Anspruch 5 oder 6, **dadurch gekennzeichnet, dass** Verbindung 1 als eine Dauerinfusion über einen Zeitraum von etwa 3 Stunden verabreicht wird.

## Revendications

1. Utilisation du composé 1 de formule A : 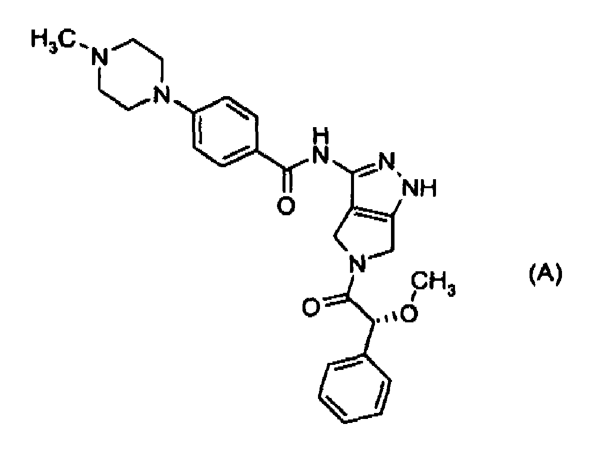 ou d'un sel pharmaceutiquement acceptable de celui-ci, et d'un vecteur ou d'un excipient pharmaceutiquement acceptables, dans la fabrication d'un médicament destiné au traitement d'un mammifère, y compris d'êtres humains, souffrant de tumeurs malignes hématopoïétiques ou solides avancées, **caractérisé en ce que** le composé 1 est perfusé par voie intraveineuse les jours 1, 8 et 15 toutes les 4 semaines, un seul jour par semaine ou toutes les deux semaines, pendant trois jours consécutifs toutes les deux semaines, pendant trois jours consécutifs la première semaine puis deux fois par semaine de la semaine 2 à la semaine 4 en cycles de 5 semaines, ou pendant 7 jours consécutifs toutes les 2 semaines.

2. Utilisation selon la revendication 1, **caractérisée en ce que** le composé 1 est perfusé par voie intraveineuse les jours 1, 8 et 15 toutes les 4 semaines en une quantité comprise entre 100 et 850 mg/m²/jour.

3. Utilisation selon la revendication 1, **caractérisée en ce que** le composé est perfusé par voie intraveineuse un seul jour par semaine ou toutes les deux semaines en une quantité de 100 à 1000 mg/m²/jour.

4. Utilisation selon la revendication 1, **caractérisée en ce que** le composé 1 est perfusé par voie intraveineuse pendant trois jours consécutifs toutes les deux semaines en une quantité de 100 à 1000 mg/m²/jour.

5. Utilisation selon la revendication 1, **caractérisée en ce que** le composé 1 est perfusé par voie intraveineuse pendant trois jours consécutifs la première semaine, puis deux fois par semaine de la semaine 2 à la semaine 4 ou de la semaine 2 à la semaine 5 en cycles de 5 semaines en une quantité comprise entre 100 et 1000 mg/m²/jour.

6. Utilisation selon la revendication 1, **caractérisée en ce que** le composé 1 est perfusé par voie intraveineuse pendant sept jours consécutifs toutes les 2 semaines en une quantité comprise entre 40 et 500 mg/m²/jour.

7. Utilisation selon la revendication 1, **caractérisée en ce que** le composé 1 est administré sous la forme d'une perfusion continue sur une période de 30 minutes à 24 heures.

8. Utilisation selon la revendication 1, **caractérisée en ce que** le composé 1 est administré sous la forme d'une perfusion continue sur une période de 1 à 24 heures.

9. Utilisation selon la revendication 2, **caractérisée en ce que** le composé 1 est administré sous la forme d'une perfusion continue sur une période d'environ 6 heures.

10. Utilisation selon la revendication 3, **caractérisée en ce que** le composé 1 est administré sous la forme d'une perfusion continue sur une période d'environ 24, 6 ou 3 heures.

11. Utilisation selon la revendication 4, **caractérisée en ce que** le composé 1 est administré sous la forme d'une perfusion continue sur une période d'environ 1 à 6 heures ou d'environ 3 heures.

12. Utilisation selon les revendications 5 ou 6, **caractérisée en ce que** le composé 1 est administré sous la forme d'une perfusion continue sur une période d'environ 3 heures.
